# EUROPÄISCHE PATENTANMELDUNG

(11) **EP 2 796 125 A1**
(43) Veröffentlichungstag der Anmeldung: **29.10.2014**
(21) Anmeldenummer: 13165574.8
(22) Anmeldetag: 26.04.2013
(51) Int. Cl.: A61H 23/02, A61F 13/20, A61H 19/00, A61B 5/00, A61H 21/00, A61F 7/00

(54) **Tamponanordnung**

(71) Anmelder: Marlafin AG, 6332 Hagendorn (CH)
(72) Erfinder: Hammen, Axel, 5426 Lengnau (CH)
(74) Vertreter: AMMANN PATENTANWÄLTE AG BERN

(57) **Zusammenfassung**

Die Tamponanordnung hat einen Tampon (2) und einen darin angeordneten Verbraucher (3). Letzterer ist mit einem ersten Ende einer Leitung (4) verbunden und mit einem zweiten Ende der Leitung (4) ist ein Gehäuse (5) verbunden. Mit dem Gehäuse (5) ist ein weiteres Gehäuse einer Stromversorgungseinheit (6) durch mechanische Verbindungsmittel lösbar verbunden, wobei das weitere Gehäuse eine elektrische Energiequelle und damit verbundene elektrische Kontakte enthält, die mit im Gehäuse (5) angeordneten elektrischen Kontakten in Berührung bringbar sind. Damit ist die Energiequelle einfach entfernbar und kann nicht durch eine unpassende Energiequelle ersetzt werden.

## Beschreibung

Die Erfindung betrifft eine Tamponanordnung mit einem in einem Tampon angeordneten, mit einem ersten Ende einer Leitung verbundenen Verbraucher und einem mit einem zweiten Ende der Leitung verbundenen Gehäuse.

Unter dem Begriff Verbraucher ist im vorliegenden Zusammenhang jegliches Element zu verstehen, das elektrische Energie verbraucht. Darunter fallen beispielsweise Vibrationserzeuger, Heizelemente oder Messelemente, insbesondere für therapeutische und diagnostische Zwecke.

Eine derartige Tamponanordnung ist im Dokument EP2360755A1 beschrieben und dient zur Bekämpfung von menstrualen Schmerzen. Die Anordnung umfasst ein in einem Tampon angeordnetes Vibrationselement und eine mit dem Tampon durch ein Kabel verbundenes Gehäuse, enthaltend eine Energiequelle und ein als Drucktaste ausgebildetes Bedienungselement, das die elektrische Verbindung zwischen der Energiequelle und dem Vibrationselement steuert. Diese Tamponanordnung wird mit einer eingebauten Batterie geliefert. Damit nach der Verwendung die Energiequelle und die übrigen Komponenten getrennt entsorgt werden können, ist in der Aussenwand des Gehäuses ein nicht wiederverschliessbarer Aufreissverschluss zum Bilden einer Öffnung zwecks Entnahme der elektrischen Energiequelle angeordnet. Somit ist bei dieser Tamponanordnung das Gehäuse und der damit zusammenhängende Tampon inklusive Vibrationselement nach dem Entfernen der Energiequelle nicht mehr verwendbar. In gewissen Fällen kann es aber wünschbar sein, mindestens das Gehäuse mit den darin angeordneten Komponenten wie beispielsweise Bedienungs-und/oder Steuerelementen wiederzuverwenden.

Ausgehend von diesem Stand der Technik liegt der Erfindung insbesondere die Aufgabe zu Grunde, eine Tamponanordnung zu schaffen, bei der die Energiequelle einfach entfernbar und nicht durch eine unpassende Energiequelle ersetzbar ist.

Diese Aufgabe wird erfindungsgemäss dadurch gelöst, dass mit dem Gehäuse ein weiteres Gehäuse durch mechanische Verbindungsmittel lösbar verbunden ist und dass das weitere Gehäuse eine elektrische Energiequelle und damit verbundene elektrische Kontakte enthält, die mit im Gehäuse angeordneten elektrischen Kontakten in Berührung bringbar sind.

Diese erfindungsgemässe Lösung hat insbesondere den Vorteil, dass die Energiequelle nach dem Gebrauch der Tamponanordnung mitsamt dem sie enthaltenden weiteren Gehäuse ohne Zuhilfenahme eines Werkzeuges vom Gehäuse trennbar ist. Ausserdem wird durch die Anordnung der Energiequelle in dem weiteren Gehäuse verhindert, dass die Energiequelle durch eine unpassende Energiequelle ersetzt wird.

Nach einer Ausführungsart der Erfindung umschliesst das weitere Gehäuse die Energiequelle derart, dass die Energiequelle nicht ohne Zerstörung des weiteren Gehäuses aus diesem entnommen werden kann. Damit ist eine typenspezifische Stromversorgungseinheit geschaffen, die nicht durch eine beliebige, möglicherweise unpassende Stromquelle ersetzbar ist.

Gemäss einer weiteren Ausführungsart enthalten die mechanischen Verbindungsmittel am Gehäuse oder am weiteren Gehäuse angeordnete Rastarme, die an ihrem freien Ende eine Rastnase aufweisen, die in eine am weiteren Gehäuse beziehungsweise am Gehäuse angeordnete Ringnut eingreifen. Diese Massnahmen ermöglichen es, das Gehäuse und das weitere Gehäuse in der Art einer Schnappverbindung miteinander zu verbinden.

Nach einer weiteren Ausführungsart sind ausgehend von der Ringnut zum die Rastarme aufweisenden Gehäuse gerichtete, geneigte Einführrampen und in einem Abstand davon entgegengesetzt geneigte Ausführrampen für die Rastnasen angeordnet. Dadurch kann gewährleistet werden, dass sowohl das Verbinden des weiteren Gehäuses mit dem Gehäuse als auch das Trennen des weiteren Gehäuses vom Gehäuse jeweils ausschliesslich in einer bestimmten relativen Position möglich ist, wobei sich diese beiden Positionen zwingend unterscheiden.

Gemäss einer anderen Ausführungsart enthalten die mechanischen Verbindungsmittel einen im Gehäuse angeordneten, durchgehenden Kanal zur Aufnahme des weiteren Gehäuses. Dadurch kann das weitere Gehäuse nach Art einer Schublade im Gehäuse angeordnet werden.

Eine weitere Ausführungsart sieht vor, dass zwischen dem Kanal und dem weiteren Gehäuse wirksame Rastnocken vorhanden sind, die eine Bewegung des weiteren Gehäuses in eine Richtung verhindern. Dadurch kann das von einer Seite her in das Gehäuse eingeführte weitere Gehäuse ausschliesslich auf der gegenüberliegenden Seite aus dem Gehäuse entnommen werden.

Nach einer weiteren Ausführungsart enthalten die mechanischen Verbindungsmittel am Gehäuse oder am weiteren Gehäuse angeordnete Bajonettflügel, die in eine am weiteren Gehäuse beziehungsweise am Gehäuse angeordnete Ausnehmung eingreifen. Eine solche Bajonettverbindung wird durch eine Längsbewegung gefolgt von einer Drehbewegung verbunden und durch eine Drehbewegung gefolgt von einer Längsbewegung getrennt.

Gemäss einer zusätzlichen Ausführungsart sind Sperrmittel vorhanden, die eine Relativdrehung zwischen dem Gehäuse und dem weiteren Gehäuse ausschliesslich in einer Richtung gestatten. Damit ist in Kombination mit der Bajonettverbindung die Möglichkeit geschaffen, dass das Verbinden des weiteren Gehäuses mit dem Gehäuse wie auch das Trennen des weiteren Gehäuses vom Gehäuse jeweils ausschliesslich in einer bestimmten relativen Position möglich ist, wobei sich diese beiden Positionen zwingend unterscheiden

Nach einer anderen Ausführungsart sind im Gehäuse und/oder im weiteren Gehäuse Biegekurven angeordnet, durch welche die im weiteren Gehäuse und/oder im Gehäuse angeordneten Kontakte bei einer Relativbewegung des weiteren Gehäuses gegenüber dem Gehäuse umgebogen werden. Damit kann eine einmal verwendete Stromversorgungseinheit für eine weitere Verwendung unbrauchbar gemacht werden.

Eine andere Möglichkeit, eine einmal verwendete Stromversorgungseinheit für eine weitere Verwendung unbrauchbar zu machen, besteht nach einer anderen Ausführungsart darin, dass mit dem weiteren Gehäuse ein Gewindering einstückig mit einer Sollbruchstelle verbunden ist, dessen Gewinde in ein am Gehäuse angeordnetes Gewinde eingreift. Nach dem Abreissen des Gewinderings fehlt am weiteren Gehäuse ein Befestigungsmittel zum Verbinden des weiteren Gehäuses mit dem Gehäuse.

Schliesslich ist nach einer weiteren Ausführungsart vorgesehen, dass im weiteren Gehäuse ein RFID-Transponder angeordnet ist. Diese Massnahme eröffnet weitere Möglichkeiten, beispielsweise zu verhindern, dass eine unpassende oder bereits verwendete Stromquelle eingesetzt wird.

Ein anderer Aspekt der Erfindung betrifft eine Stromversorgungseinheit. Diese umfasst eine im weiteren Gehäuse angeordnete elektrische Energiequelle und damit verbundene elektrische Kontakte sowie mechanische Verbindungsmittel zum Verbinden der Stromversorgungseinheit mit dem Gehäuse einer Tamponanordnung.

Ausführungsbeispiele der Erfindung werden nachstehend unter Bezugnahme auf die angefügten Zeichnungen beispielsweise näher beschrieben. Es zeigt
- Figur 1: eine schematische Gesamtansicht einer erfindungsgemässen Tamponanordnung;
- Figur 2: eine schematische Schnittansicht eines ersten Ausführungsbeispiels einer Stromversorgungseinheit vor dem Verbinden mit dem Gehäuse;
- Figur 3: eine Schnittansicht des ersten Ausführungsbeispiels verbunden mit dem Gehäuse in kontaktiertem Zustand;
- Figur 4: eine Schnittansicht des ersten Ausführungsbeispiels verbunden mit dem Gehäuse, jedoch mit getrennten Kontakten;
- Figur 5: eine Schnittansicht des ersten Ausführungsbeispiels nach dem Trennen vom Gehäuse;
- Figur 6: eine perspektivische Ansicht des Verbindungsbereichs des Gehäuses für das erste Ausführungsbeispiel der Stromversorgungseinheit;
- Figur 7: eine schematische Schnittansicht eines zweiten Ausführungsbeispiels einer Stromversorgungseinheit vor dem Verbinden mit einem passenden Gehäuse;
- Figur 8: eine Schnittansicht der Stromversorgungseinheit nach Figur 7 verbunden mit dem Gehäuse in kontaktiertem Zustand;
- Figur 9: eine Schnittansicht der Stromversorgungseinheit nach dem Trennen vom Gehäuse;
- Figur 10: eine schematische Schnittansicht eines dritten Ausführungsbeispiels einer mit einem passenden Gehäuse verbundenen Stromversorgungseinheit;
- Figur 11: eine gegenüber der Darstellung von Figur 10 um 90 Grad gedrehte, schematische Schnittansicht;
- Figur 12: eine Schnittansicht entsprechend Figur 10 der Stromversorgungseinheit nach der Entnahme vom Gehäuse;
- Figur 13: eine schematische Schnittansicht eines vierten Ausführungsbeispiels einer Stromversorgungseinheit;
- Figur 14: eine schematische Schnittansicht einer Stromversorgungseinheit gemäss Figur 13, verbunden mit einem entsprechenden Gehäuse und
- Figuren 15 und 16: schematische Schnittansichten der Stromversorgungseinheit nach Figur 13 nach dem Trennen vom Gehäuse.

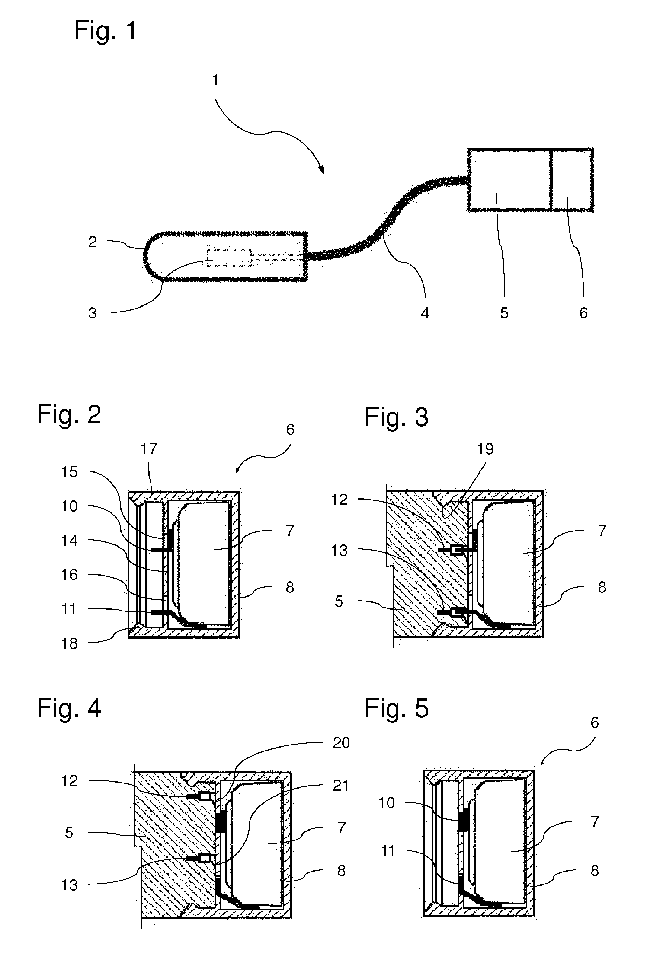

Die in Figur 1 schematisch dargestellte Tamponanordnung 1 umfasst im Wesentlichen einen Tampon 2 mit bzw. aus saugfähigem Material, mit dem ein Vibrationserzeuger 3 verbunden ist, derart, dass im Betrieb der ganze Tampon 2 in Schwingungen versetzt wird. Ein Kabel 4 versorgt den Vibrationserzeuger 3 mit elektrischer Energie und gleichzeitig als Rückzugsmittel zum Zurückziehen des Tampons 2 aus einer Körperöffnung dienen kann. Selbstverständlich kann auch ein konventioneller Rückholfaden vorgesehen sein. An dem vom Tampon 2 abgewandten Ende des Kabels 4 ist ein Gehäuse 5 angeordnet, das seinerseits mit einer Stromversorgungseinheit 6 lösbar verbunden ist.

Figur 2 zeigt in einer schematischen Schnittansicht ein erstes Ausführungsbeispiel der Stromversorgungseinheit 6. Mit 7 ist eine Stromquelle bezeichnet, die als Batterie ausgebildet sein kann und in den dargestellten Beispielen die Gestalt einer Knopfzelle hat. Es ist aber auch denkbar, einen wiederaufladbaren Akkumulator als Stromquelle zu verwenden. Die Stromquelle 7 ist in einem weiteren Gehäuse 8 derart eingeschlossen, dass sie daraus nicht ohne Zerstörung des weiteren Gehäuses 8 entnehmbar ist. Dies kann beispielsweise erreicht werden, indem Teile des weiteren Gehäuses 8 nach dem Einbringen der Stromquelle 7 untrennbar verbunden werden, etwa durch Kleben oder Schweissen, oder indem das weitere Gehäuse 8 nach dem Einbringen der Stromquelle 7 thermisch verformt wird oder indem die Stromquelle 7 im weiteren Gehäuse 8 beispielsweise mit einer Kunststoffmasse eingegossen wird oder indem das weitere Gehäuse durch Umgiessen der Stromquelle 7, beispielsweise mit einer Kunststoffmasse gebildet wird. Zwei mit entsprechenden Polen der Stromquelle 7 elektrisch verbundene Kontakte 10, 11 sind durch entsprechende Öffnungen 15, 16 in einer Wand 14 des weiteren Gehäuses herausgeführt.

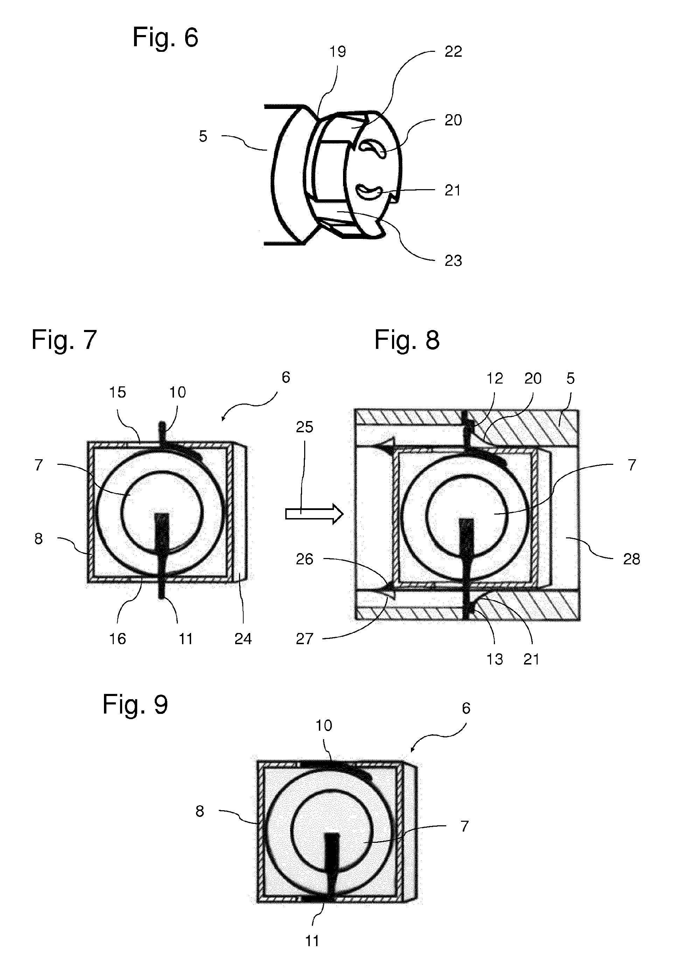

Zur lösbaren Befestigung des weiteren Gehäuses 8 am Gehäuse 5 sind am weiteren Gehäuse 8 Rastarme 17 vorgesehen, die an ihrem Ende eine Rastnase 18 tragen, die in eine Ringnut 19 (siehe Figuren 3 und 6) des Gehäuses 5 eingreifen. Die Dimensionen und Elastizität der Rastarme 17 und die Geometrie der Rastnasen 18 sind dabei so gewählt, dass das weitere Gehäuse 8 nicht in jeder beliebigen relativen Drehlage auf das Gehäuse 5 gesteckt werden kann, sondern nur in einer einzigen Position, welche durch Einführrampen 22 definiert ist, wie sie in Figur 6 dargestellt sind. Weiter sind die Dimensionen und Elastizität der Rastarme 17 und die Geometrie der Rastnasen 18 so gewählt, dass das weitere Gehäuse 8 nach dem Aufstecken auf das Gehäuse 5 nicht in der entgegengesetzten Richtung wieder abgezogen werden kann. Vielmehr müssen zum Abnehmen des weiteren Gehäuses 8 vom Gehäuse 5 diese beiden Gehäuse 5, 8 gegeneinander verdreht werden, bis die Rastnasen 18 mit Ausführrampen 23 fluchten, wie sie in Figur 6 dargestellt sind. Damit beispielsweise bei einer Ausführungsart mit zwei Rastarmen 17 das weitere Gehäuse 8 nicht in zwei um 180 Grad verdrehten Positionen ankuppelbar ist, was zu einer falschen Polarität oder Beschädigung der Kontakte führen könnte, sind die beiden Rastarme 17 in einem Winkelabstand von etwas weniger als 180 Grad angeordnet. Entsprechend kann bei drei oder vier Rastarmen 17 deren Winkelabstand unregelmässig gestaltet werden, so dass die Rastame 17 nur in einer einzigen Position in die Einführrampen 22 passen.

Figur 3 zeigt die auf die vorangehend beschriebene Weise mechanisch miteinander verbundenen Gehäuse 5 und 8 und darüber hinaus auch elektrische Verbindungen, indem die genannten Kontakte 10, 11 mit im Gehäuse 5 vorhandenen Kontakten 12, 13 verbunden sind. In den Figuren 2 bis 5, 7 bis 14 und 16 ist deutlich sichtbar, dass die im weiteren Gehäuse 8 angeordneten Kontakte 10 und 11 mit der Stromquelle 7 verbunden sind. Selbstverständlich sind die im Gehäuse 5 angeordneten Kontakte 12 und 13 mit Leitern verbunden, welche den Strom mittelbar oder unmittelbar zum Vibrationserzeuger 3 führen. Diese Leiter sind jedoch in den Zeichnungsfiguren nicht dargestellt. Die Kontaktpaare 10 und 12 beziehungsweise 11 und 13 sind hier als Steckverbindungen dargestellt, es sind aber auch andere Verbindungsarten denkbar. Die im vorangehenden Abschnitt beschriebene Art der mechanischen Verbindung und Trennung des weiteren Gehäuses 8 vom Gehäuse 5 hat einerseits den Vorteil, dass die Gehäuse 5 und 8 nur in einer solchen Relativlage miteinander verbindbar sind, dass auch die genannten Kontaktpaare 10 und 12 beziehungsweise 11 und 13 lagemässig übereinstimmen und somit gleichzeitig der elektrische Kontakt hergestellt wird. Andererseits müssen die beiden Gehäuse 5, 8 zum Trennen zuerst gegeneinander verdreht werden, bevor sie axial auseinander gezogen werden können, was zur Folge hat, dass die Kontaktpaare getrennt werden. In den Figuren 4 und 6 des dargestellten Beispiels sind im Gehäuse 5 Biegekurven 20 und 21 angeordnet, welche bewirken, dass beim Verdrehen der Gehäuse 5, 8 die Kontakte 10, 11 getrennt und verbogen werden. In Figur 5 ist zu sehen, wie die Kontakte 10, 11 im abgenommenen weiteren Gehäuse 8 derart gebogen sind, so dass sie parallel zur Wand 14 liegen. Vorteilhaft sind die Öffnungen 15, 16 derart gestaltet, dass sie die umgebogenen Kontakte 10, 11 praktisch lückenlos aufnehmen, wodurch verhindert wird, dass die Kontakte wieder gerade gebogen und die Stromversorgungseinheit 6 ein weiteres Mal verwendet wird. Alternativ oder zusätzlich können die Kontakte 10, 11 aus einem solchen Material gefertigt sein, dass sie beim Versuch, sie in ihre ursprüngliche Lage zurück zu biegen, brechen. Um eine relative Drehung des weiteren Gehäuses 8 gegenüber dem Gehäuse 5 in der falschen Richtung zu verhindern, kann die Ringnut 19 unterbrochen sein oder es können in ihr Sperrnocken (nicht dargestellt) angeordnet sein.

Das Prinzip, dass beim Abnehmen der Stromversorgungseinheit 6 Kontakte verbogen werden, kann auch kinematisch umgekehrt werden, so dass beim Abnehmen die Kontakte im Gehäuse 5 verbogen werden und dieses damit unbrauchbar wird. Ausserdem können die beiden genannten Varianten auch kombiniert werden, derart, dass beim Abnehmen der Stromversorgungseinheit 6 die Kontakte in beiden Gehäusen und 8 verbogen werden, wodurch sowohl das Gehäuse als auch das die Stromquelle enthaltende weitere Gehäuse 8 für eine weitere Verwendung unbrauchbar werden. Ebenso können in kinematischer Umkehr die Rastarme 17 am Gehäuse 5 und die Ringnut 19 sowie die Einführrampen 22 und Ausführrampen 23 am weiteren Gehäuse 8 angeordnet sein.

Die Figuren 7 bis 9 zeigen eine andere Ausführungsart der Stromversorgungseinheit 6 und des dazu passenden Gehäuses 5. Auch bei dieser Ausführungsart ist die Stromquelle 7 im weiteren Gehäuse 8 derart eingeschlossen, dass sie daraus nicht ohne Zerstörung des weiteren Gehäuses 8 entnehmbar ist. Das weitere Gehäuse 8 hat bei dieser Ausführungsart eine viereckige Form und die Kontakte 10 und 11 ragen vor der Benutzung, dargestellt in Figur 7 und während der Benutzung, dargestellt in Figur 8 auf gegenüberliegenden Seiten aus dem weiteren Gehäuse 8 heraus. Das Gehäuse 5 weist einen durchgehenden Kanal 28 für die Stromversorgungseinheit 6 auf und letztere wird in Richtung des Pfeils 25 so weit eingeführt, bis ihre Kontakte 10 und 11 an den im Gehäuse 5 angeordneten Kontakten 12, 13 anliegen. Das Erreichen dieser in Figur 8 dargestellten Position wird durch einen durch die Berührung der Kontaktpaare 10 und 12 beziehungsweise 11 und 13 verursachten erhöhten mechanischen Widerstand taktil angezeigt. Rastnocken 26 sorgen dafür, dass das Gehäuse 8 sich nicht entgegen der Richtung des Pfeils 25 zurück bewegen kann und dass der Kontakt zwischen den Kontaktpaaren aufrecht erhalten wird. Beim Einführen des Gehäuses 8 weichen die Rastnocken 26 in Ausnehmungen 27 aus. Umgekehrt können Rastnocken auch am weiteren Gehäuse 8 vorgesehen sein und in Ausnehmungen einrasten, die an Innenwänden des Kanals 28 vorgesehen sind. Ein am Gehäuse 8 angeordneter Einführkeil 24 erleichtert das richtige, insbesondere polrichtige Einführen der Stromversorgungseinheit 6 in das Gehäuse 8. Zusätzlich kann beispielsweise mit zwischen den Gehäusen 5 und 8 angeordneten, nicht dargestellten Rippen-Nuten-Paaren, die beispielsweise auf gegenüberliegenden Seiten des Kanals 28 auf unterschiedlichen Höhen angeordnet oder unterschiedlich breit sind ein falsches Einlegen der Stromversorgungseinheit 6 verhindert werden. Auch die Kontakte 10 und 11 selbst können das lagerichtige Einführen des weiteren Gehäuses 8 in den Kanal 28 unterstützen.

In einer zusätzlichen Variante dieser Ausführungsart können zwei Paare von Rastnocken 26 in Richtung des Pfeils 25 hintereinander angeordnet sein, wodurch zwei Positionen des weiteren Gehäuses 8 im Gehäuse 5 definiert werden. In einer ersten Position, die dem Auslieferungszustand der Tamponanordnung 1 entsprechen kann, sitzt die Stromversorgungseinheit 6 im Gehäuse 5, ohne dass die Kontaktpaare 10, 12 und 11, 13 sich berühren. Dabei wird die Stromversorgungseinheit 6 durch das erste Rastnockenpaar an einem Rückzug aus dem Gehäuse 5 gehindert und beispielsweise durch das zweite, in Bewegungsrichtung nachgeordnete Rastnockenpaar im Kanal 28 reibschlüssig Festgehalten. Zum Einschalten der Tamponanordnung 1 wird die Stromversorgungseinheit 6 in Pfeilrichtung weiter bewegt, bis sich die Kontaktpaare mit dem nötigen Kontaktdruck berühren und wird durch das weitere Nockenpaar in dieser Position festgehalten.

Zum Entnehmen der Stromversorgungseinheit 6 aus dem Gehäuse 5 wird die Stromversorgungseinheit 6 in Richtung des Pfeils 25 weiter bewegt, wobei die Kontaktpaare dieser Weiterbewegung einen erhöhten mechanischen Widerstand entgegensetzen. Gegebenenfalls kann dieser Widerstand beispielsweise mit Hilfe weiterer, nicht dargestellter Rastmittel erhöht werden oder es können manuell zu betätigende Sperrmittel wie ein Druckknopf oder ein herausziehbarer Stift vorgesehen sein. Ähnlich wie bei der vorangehend beschriebenen Ausführungsart trennen sich während der Weiterbewegung die Kontaktpaare und die Kontakte 10 und 11 werden anschliessend durch Biegekurven 20 und 21 umgebogen.

Figur 9 zeigt die Stromversorgungseinheit 6 nach der Entnahme aus dem Gehäuse 5. Ähnlich wie bei der vorangehend beschriebenen Ausführungsart sind die flachgelegten Kontakte 10, 11 in Öffnungen 15, 16 des weiteren Gehäuses 8 gedrückt, so dass sie nicht wieder gerade gebogen werden können. Ein wieder gerade Biegen kann auch hier zusätzlich durch die Wahl des Werkstoffes der Kontakte erschwert oder verhindert werden. Auch bei dieser Ausführungsart kann die Anordnung der Kontakte so getroffen werden, dass beim Entnehmen der Stromversorgungseinheit 6 die Kontakte im Gehäuse 5 oder in beiden Gehäusen 5 und 8 umgebogen werden.

Um das Ausstossen der verbrauchten Stromversorgungseinheit 6 aus dem Gehäuse 5 zu erleichtern, kann am weiteren Gehäuse 8 an der dem Einführkeil 24 gegenüberliegenden Seite ein Ansatz oder eine Verlängerung vorhanden sein, die in der in Figur 8 dargestellten Gebrauchssituation links aus dem Gehäuse 5 heraus ragt.

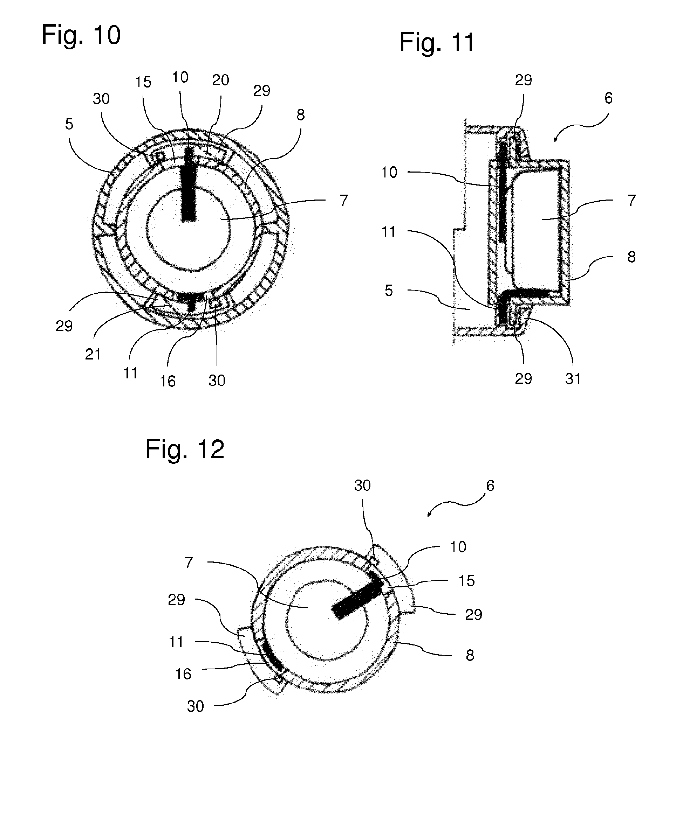

In den Figuren 10 bis 12 ist eine dritte Ausführungsart der Stromversorgungseinheit 6 und des entsprechenden Gehäuses 5 dargestellt. Auch bei dieser Ausführungsart ist die Stromquelle 7 derart im weiteren Gehäuse 8 eingeschlossen, dass sie nicht ohne Zerstörung des weiteren Gehäuses 8 aus diesem entnommen werden kann. Das weitere Gehäuse 8 hat hier einen runden Querschnitt (siehe Figuren 10 und 12) und die Kontakte 10 und 11 ragen radial aus dem weiteren Gehäuse 8 heraus. Ausserdem sind am weiteren Gehäuse 8 Bajonettflügel 29 angeformt, die in entsprechende Ausnehmungen im Gehäuse 5 passen. Zum Einsetzen und Entnehmen der Stromversorgungseinheit 6 sind in einer stirnseitigen Gehäusewand 31 des Gehäuses 5 Ausnehmungen angeordnet, durch welche die Bajonettflügel 29 passen. Diese Ausnehmungen sind im dargestellten Beispiel so angeordnet, dass in der relativen Drehlage, in der die Stromversorgungseinheit eingesetzt wird, noch kein Kontakt zwischen den Kontakten 10, 11 der Stromversorgungseinheit 6 und den hier nicht sichtbaren Kontakten im Gehäuse 5 stattfindet. Erst wenn die Stromversorgungseinheit 6 in der Darstellung nach Figur 10 im Uhrzeigersinn in die dargestellte Lage gedreht wird, findet der Kontakt statt. Damit ist, ähnlich wie anhand des vorangehenden Beispiels beschrieben, eine zusätzliche Schaltfunktion geschaffen. Nicht sichtbare Sperrnocken, die in Rastöffnungen 30 der Bajonettflügel eingreifen, verhindern in dieser Position das Zurückdrehen der Stromversorgungseinheit 6. Wird die Stromversorgungseinheit 6 trotzdem gewaltsam zurück gedreht, werden die Sperrnocken beschädigt, so dass das Gehäuse 5 dadurch für eine weitere Verwendung unbrauchbar gemacht wird.

Zum Entnehmen der Stromversorgungseinheit 6 wird diese ausgehend von der in Figur 10 dargestellten Position im Uhrzeigersinn weiter gedreht, wobei zunächst die Kontaktpaare getrennt und danach die Kontakte 10, 11 durch die Biegekurven 20, 21 umgebogen werden, derart dass sie in die Öffnungen 15 bzw. 16 eingedrückt werden und damit die Stromversorgungseinheit für eine weitere Verwendung unbrauchbar machen.

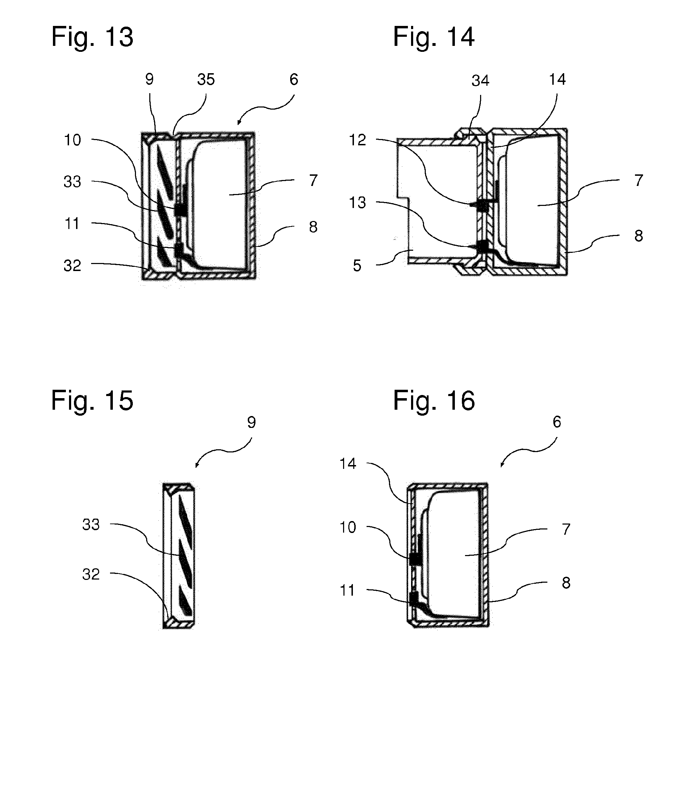

In den Figuren 13 bis 16 ist ein viertes Ausführungsbeispiel der Energieversorgungseinheit 6 dargestellt. Auch bei dieser Ausführungsart kann die Stromquelle 7 im weiteren Gehäuse 8 derart eingeschlossen sein, dass sie daraus nicht ohne Zerstörung des weiteren Gehäuses 8 entnehmbar ist. Figur 13 zeigt die Energieversorgungseinheit 6 vor dem Verbinden mit dem Gehäuse 5, während in Figur 14 die Energieversorgungseinheit 6 im mit dem Gehäuse 5 verbundenen Zustand zeigt. Am weiteren Gehäuse 8 ist ein Abreissring 9 einstückig angeformt, derart, dass zwischen dem weiteren Gehäuse 8 und dem Abreissring 9 eine Sollbruchstelle 35 gebildet ist. Im Abreissring 9 ist ein Gewinde 33 vorhanden, das beim Ansetzen der Energieversorgungseinheit 6 an das Gehäuse 5 mit einem entsprechenden, am Gehäuse 5 angeordneten Gewinde 34 in Eingriff gebracht wird. Eine am Abreissring 9 angeformte umlaufende innere Rastrippe 32 wird ausserdem beim Montieren durch das Gewinde 34 gedehnt, federt nach dem Passieren des Gewindes 34 zurück und verhindert das Abschrauben der Stromversorgungseinheit 6. Gegebenenfalls können zusätzliche Mittel zum Verhindern des Zurückdrehens des Abreissrings 9 vorgesehen werden, wie beispielsweise kleine Zähne am innenumfang des Abreissrings 9.

Die Kontakte 10 und 11 an der Stromversorgungseinheit 6 sowie auch die Kontakte 12 und 13 am Gehäuse 5 weisen in diesem Beispiel parallele Kontaktflächen auf, die beim Anschrauben der Stromversorgungseinheit schleifend miteinander in Berührung kommen. Dies ermöglicht eine Schaltfunktion der Kontakte, indem die Stromversorgungseinheit 9 beispielsweise für die Auslieferung der Tamponanordnung 1 nur so weit angeschraubt wird, dass die Rastrippe 32 das Abnehmen der Stromversorgungseinheit verhindert, aber die Kontaktpaare 10, 12 und 11, 13 noch nicht miteinander verbunden sind. Zur Inbetriebnahme der Tamponanordnung wird die Stromversorgungseinheit weiter gedreht, bis der Kontakt hergestellt ist. Diese Position kann durch Markierungen am Gehäuse 5 und am weiteren Gehäuse 8 angezeigt werden, aber auch taktil, beispielsweise indem die Wand 14 des weiteren Gehäuses 8 stirnseitig am Gehäuse 5 ansteht.

Zum Ausschalten der Tamponanordnung 1 und gleichzeitig zum Entfernen der Stromversorgungseinheit 6 wird letztere in einem das Gewinde lösenden Sinne zurückgedreht, wobei aber der Abreissring 9 mit der Rastrippe 32 an den Gewindegängen 34 des Gehäuses 5 ansteht und so am Gehäuse 5 festgehalten wird. Bei fortgesetzter Drehung am weiteren Gehäuse bricht die Sollbruchstelle 35. Sollte in unerwünschter Weise die Stromversorgungseinheit ausgehend von der in Figur 14 dargestellten Position im das Gewinde anziehenden Sinne weiter gedreht werden, wird der Abreissring 9 in der Darstellung nach Figur 14 weiter nach links bewegt, was zunächst zur Erhöhung des Kontaktdrucks der Kontaktpaare 10, 12 und 11, 13 führt und dann dazu, dass das weitere Gehäuse 8 dieser Bewegung nicht weiter folgen kann. Folglich wird auch in diesem Fall der Abreissring 9 vom weiteren Gehäuse 8 getrennt und das weitere Gehäuse 8 mitsamt der darin enthaltenen Stromquelle 7 kann nicht wiederverwendet werden. Vor dem Aufsetzen einer neuen Stromversorgungseinheit 6 wird der abgetrennte Abreissring 9 auf der dem Gewinde 34 entgegengesetzten Seite des Gehäuses 5 abgestreift.

Bei allen vorangehend und nachstehend beschriebenen Ausführungsbeispielen kann das Gehäuse 5 und/oder das weitere Gehäuse 8 Befehlsgeräte wie Schalter, beispielsweise Druck- Dreh- oder Kippschalter, Meldegeräte wie Signallampen oder Leuchtdioden und Steuereinrichtungen wie integrierte Schaltungen enthalten.

In einer fünften, nicht zeichnerisch dargestellten Ausführungsart enthält das weitere Gehäuse einen RFID (radio-frequency identification) Transponder und im Gehäuse ist Kommunikationsgerät eingebaut, das mit dem Transponder und einer Steuerelektronik kommuniziert. Damit besteht die Möglichkeit, die Verwendung einer passenden Stromversorgungseinheit zu überwachen und zu steuern und insbesondere auch die mehrfache Verwendung ein und derselben Stromversorgungseinheit zu verhindern. Im letztgenannten Fall kann die Steuerelektronik über das Kommunikationsgerät ein Signal, einen so genannten "Kill-Code" an den Transponder senden, der damit seine Kennung verliert und in der Folge von der Steuerelektronik nicht mehr erkannt wird. Zusätzlich eröffnet eine solche Anordnung die Möglichkeit, mittels der Steuerelektronik festzustellen, wie viele Stromquellen bereits mit der Tamponanordnung verwendet wurden.

Die Erfindung ist nicht auf die dargestellten und beschriebenen Ausführungsbeispiele beschränkt, sondern umfasst auch Merkmalskombinationen aus verschiedenen Ausführungsbeispielen. So kann zum Beispiel ein anhand der fünften Ausführungsart beschriebener Transponder auch in den anderen vier Ausführungsarten verwendet werden. Darüber hinaus sind weitere Kombinationen der beschriebenen mechanischen Verbindungsmittel mit den beschriebenen elektrischen Verbindungsmittel möglich.

**Bezugszeichenliste**

| | | | |
|---|---|---|---|
| 1 | Tamponanordnung | 31 | Gehäusewand |
| 2 | Tampon | 32 | Rastrippe |
| 3 | Vibrationserzeuger | 33 | Gewinde |
| 4 | Kabel | 34 | Gewinde |
| 5 | Gehäuse | 35 | Sollbruchstelle |
| 6 | Stromversorgungseinheit | | |
| 7 | Stromquelle | | |
| 8 | Weiteres Gehäuse | | |
| 9 | Abreissring | | |
| 10 | Stromquellenkontakt | | |
| 11 | Stromquellenkontakt | | |
| 12 | Gehäusekontakt | | |
| 13 | Gehäusekontakt | | |
| 14 | Wand | | |
| 15 | Öffnung | | |
| 16 | Öffnung | | |
| 17 | Rastarm | | |
| 18 | Rastnase | | |
| 19 | Ringnut | | |
| 20 | Biegekurve | | |
| 21 | Biegekurve | | |
| 22 | Einführrampe | | |
| 23 | Ausführrampe | | |
| 24 | Einführkeil | | |
| 25 | Pfeil | | |
| 26 | Rastnocken | | |
| 27 | Ausnehmung | | |
| 28 | Kanal | | |
| 29 | Bajonettflügel | | |
| 30 | Rastöffnung | | |

## Patentansprüche

1. Tamponanordnung mit einem in einem Tampon (2) angeordneten, mit einem ersten Ende einer Leitung (4) verbundenen Verbraucher (3) und einem mit einem zweiten Ende der Leitung (4) verbundenen Gehäuse (5), **dadurch gekennzeichnet, dass** mit dem Gehäuse (5) ein weiteres Gehäuse (8) durch mechanische Verbindungsmittel (17, 18, 19; 28; 29; 9) lösbar verbunden ist und dass das weitere Gehäuse (8) eine elektrische Energiequelle (7) und damit verbundene elektrische Kontakte (10, 11) enthält, die mit im Gehäuse (5) angeordneten elektrischen Kontakten (12, 13) in Berührung bringbar sind.

2. Tamponanordnung nach Anspruch 1, **dadurch gekennzeichnet, dass** das weitere Gehäuse (8) die Energiequelle (7) derart umschliesst, dass die Energiequelle (7) nicht ohne Zerstörung des weiteren Gehäuses (8) aus diesem entnommen werden kann.

3. Tamponanordnung nach einem der vorangehenden Ansprüche, **dadurch gekennzeichnet, dass** die mechanischen Verbindungsmittel am Gehäuse (5) oder am weiteren Gehäuse (8) angeordnete Rastarme (17) enthalten, die an ihrem freien Ende eine Rastnase (18) aufweisen, die in eine am weiteren Gehäuse (8) beziehungsweise am Gehäuse (5) angeordnete Ringnut (19) eingreifen. (Fig. 2 bis 6)

4. Tamponanordnung nach Anspruch 3, **dadurch gekennzeichnet, dass** ausgehend von der Ringnut (19) zum die Rastarme (17) aufweisenden Gehäuse (5; 8) gerichtete, geneigte Einführrampen (22) und in einem Abstand davon entgegengesetzt geneigte Ausführrampen (23) für die Rastnasen (18) angeordnet sind. (Fig. 6)

5. Tamponanordnung nach einem der Ansprüche 1 bis 2, **dadurch gekennzeichnet, dass** die mechanischen Verbindungsmittel einen im Gehäuse (5) angeordneten, durchgehenden Kanal (28) zur Aufnahme des weiteren Gehäuses (8) enthalten. (Fig. 7 bis 9)

6. Tamponanordnung nach Anspruch 5, **dadurch gekennzeichnet, dass** zwischen dem Kanal (28) und dem weiteren Gehäuse (8) wirksame Rastnocken (26) vorhanden sind, die eine Bewegung des weiteren Gehäuses (8) in eine Richtung verhindern. (Fig. 8)

7. Tamponanordnung nach einem der Ansprüche 1 bis 2, **dadurch gekennzeichnet, dass** die mechanischen Verbindungsmittel am Gehäuse (5) oder am weiteren Gehäuse (8) angeordnete Bajonettflügel (29) enthalten, die in eine am weiteren Gehäuse (8) beziehungsweise am Gehäuse (5) angeordnete Ausnehmung eingreifen. (Fig. 10 bis 12)

8. Tamponanordnung nach Anspruch 7, **dadurch gekennzeichnet, dass** Sperrmittel (30) vorhanden sind, die eine Relativdrehung zwischen dem Gehäuse (5) und dem weiteren Gehäuse (8) ausschliesslich in einer Richtung gestatten. (Fig. 10, 12)

9. Tamponanordnung nach einem der vorangehenden Ansprüche, **dadurch gekennzeichnet, dass** im Gehäuse (5) und/oder im weiteren Gehäuse (8) Biegekurven (20, 21) angeordnet sind, durch welche die im weiteren Gehäuse (8) und/oder im Gehäuse (5) angeordneten Kontakte (10, 11, 12, 13) bei einer Relativbewegung des weiteren Gehäuses (8) gegenüber dem Gehäuse (5) umgebogen werden. (Fig 2 bis 12)

10. Tamponanordnung nach einem der Ansprüche 1 bis 2, **dadurch gekennzeichnet, dass** mit dem weiteren Gehäuse (8) ein Gewindering (9) einstückig mit einer Sollbruchstelle (35) verbunden ist, dessen Gewinde (33) in ein am Gehäuse (5) angeordnetes Gewinde eingreift. (Fig. 13 bis 16)

11. Tamponanordnung nach einem der vorangehenden Ansprüche, **dadurch gekennzeichnet, dass** im weiteren Gehäuse (8) ein RFID-Transponder angeordnet ist.

12. Stromversorgungseinheit (6) für eine Tamponanordnung nach einem der vorangehenden Ansprüche, umfassend eine im weiteren Gehäuse (8) angeordnete elektrische Energiequelle (7) und damit verbundene elektrische Kontakte (10, 11) sowie mechanische Verbindungsmittel (17, 18, 19; 28; 29; 9) zum Verbinden der Stromversorgungseinheit mit dem Gehäuse (5) einer Tamponanordnung (1).
